# FASCICULE DE BREVET EUROPEEN

(11) **EP 4 331 506 B1**
(45) Date de publication et mention de la délivrance du brevet: **22.01.2025**
(21) Numéro de dépôt: 23194927.2
(22) Date de dépôt: 01.09.2023
(51) Int. Cl.: A61B 17/122, A61B 17/128, A61B 17/00, A61B 90/00

(54) **DISPOSITIF POUR LIGATURE DE TISSU**
VORRICHTUNG ZUM ABBINDEN VON GEWEBE
DEVICE FOR LIGATING TISSUE

(30) Priorité: 03.09.2022 FR 2208854
(43) Date de publication de la demande: 06.03.2024
(73) Titulaire: Kada, Abdelkrim, 07200 Saint Etienne de Fontbellon (FR)
(72) Inventeur: Kada, Abdelkrim, 07200 Saint Etienne de Fontbellon (FR)

(56) Documents cités:
- FR-A1- 3 117 327
- US-A1- 2012 116 419
- US-A1- 2019 105 046

## Description

Selon les modes de réalisation, la présente invention concerne un dispositif pour la fermeture ou la ligature de tissu au niveau de la paroi interne d'un organe creux comme le tube digestif ou d'autres lumières corporelles et organes creux qui est relativement simple à fabriquer et à utiliser.

Les exemples des dispositifs disponibles disposent d'outils de type: « *Endoloop*^{®} » ou « *Polyloop*^{®} », avec positionnement difficile en raison d'une souplesse trop importante de sa boucle et un système de serrage et de séparation complexe.

Le document FR-3 117 327 A concerne un dispositif amélioré qui comprend un premier et un second bras, un fil de serrage reliant les deux extrémités distales des premier et second bras, un fil souple de commande passant à travers une fente, ledit fil souple de commande présentant à son extrémité une boucle entourant ledit fil de serrage, une saillie permettant de retenir la boucle et permettant la fixation du fil de serrage en position de traction et un fil rigide de commande positionné dans une encoche. L'espace entre les bras n'est toutefois pas dégagé.

La présente invention vise donc à remédier à ces inconvénients : avoir un outil simple, qui s'utilise dans le canal de travail d'un endoscope.

Ce dispositif possède deux bras rigides permettant son positionnement facilement autour de la lésion ou la zone de tissu cible avec une force de serrage prédéfinie permettant la fermeture ou la ligature du tissu cible de façon efficace et durable, il s'utilise par simple introduction dans le canal de travail de l'endoscope.

Ce dispositif comprend un élément tubulaire 1 allongé, flexible, utilisé à travers un canal de travail d'un endoscope dont une extrémité proximale reste externe au corps, accessible à un utilisateur avec manette (poignée de contrôle) tandis qu'une extrémité distale 2 de l'élément flexible est insérée dans l'organe creux après son introduction dans le canal de travail de l'endoscope jusqu'à un emplacement adjacent au tissu cible à fermer ou à ligaturer. Ledit dispositif comprend aussi deux fils de commande, un fil 9 souple de commande et un fil 13 rigide de commande s'étendant à travers l'élément flexible 1, les deux fils étant couplés de manière amovible au dispositif. Ledit dispositif peut être en matière isolante non conductrice du courant électrique (type plastique), par exemple dans le cas de résection de tissu ligaturé avec un courant de section coagulation, ou en matière conductrice (du courant électrique), par exemple dans le cas de résection de tissu ligaturé à froid ou encore dans le cas d'utilisation du dispositif uniquement pour la fermeture du tissu.

Ce dispositif comprend un premier 3 et un second 4 bras d'une longueur prédéfinie, l'extrémité distale du second bras 4 est reliée par un fil 5 souple de serrage avec une longueur prédéfinie, ledit fil 5 souple de serrage pouvant être en matière prédéfinie résorbable ou non résorbable. L'autre extrémité du fil 5 souple de serrage est fixée sur le bord externe 6 de l'extrémité distale du premier bras 3, une boucle 7 dudit fil 5 souple de serrage passe du coté externe dans la fente 8 de l'extrémité distale du premier bras 3.

Le fil 9 souple passe dans une fente10 au niveau de la partie proximale du dispositif. Ledit fil 9 passe ensuite dans la boucle 7 du fil 5. Ledit fil 9 présente à son extrémité une boucle 11 de taille prédéfinie. La boucle 11 de l'extrémité du fil 9 souple est retenue sur une saillie 12 à l'extrémité proximale du dispositif.

La partie proximale 18 du dispositif est fixée par l'extrémité 17 distale du fil 13 rigide de commande positionnée dans une liaison fragile 14. La saillie 15 de forme courbée retient l'extrémité 17 distale du fil 13 rigide de commande dans la liaison fragile qui échoue lorsque le dispositif est complètement sorti de l'extrémité distale 2 de l'élément flexible 1 pour séparer le dispositif de l'attache du fil 13 rigide de commande. Un actionneur à l'extrémité proximale de l'élément flexible 1 couplé au fil 13 rigide de commande et configuré pour déplacer le dispositif dans l'extrémité de l'élément flexible 1 jusqu'à la sortie du premier 3 et second 4 bras du dispositif permettant une position ouverte et son retrait dans l'extrémité distale de l'élément flexible 1 pour une configuration fermée. Le premier 3 et le second 4 bras du dispositif présentent une angulation 16 proximale prédéfinie permettant l'écartement des deux extrémités du premier 3 et du second 4 bras du dispositif une fois sorti de l'extrémité distale de l'élément flexible 1. Dans la configuration ouverte, les extrémités distales des premier 3 et second 4 bras du dispositif sont séparées, permettant de positionner le dispositif sur la zone de tissu cible à saisir. Dans une variante des modes de réalisation les premiers 3 et second 4 bras du dispositif contiennent des griffes en nombre prédéfini orientées face interne permettant l'accrochage du premier 3 et second 4 bras au tissu cible.

### Fonctionnement du dispositif :

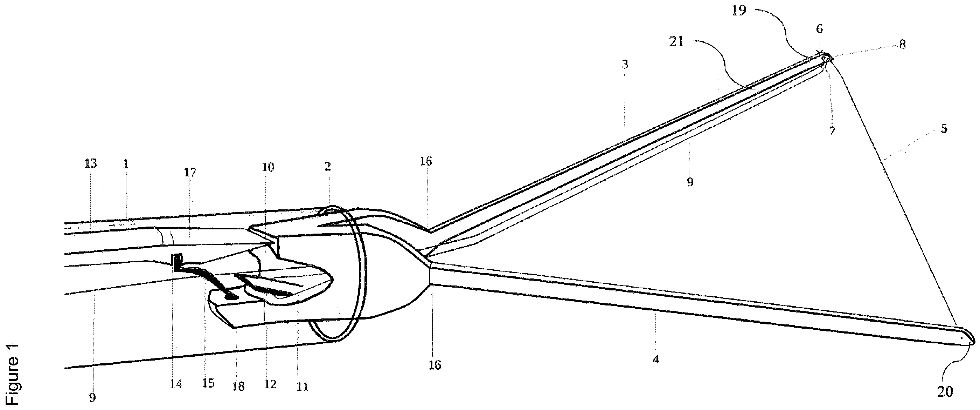

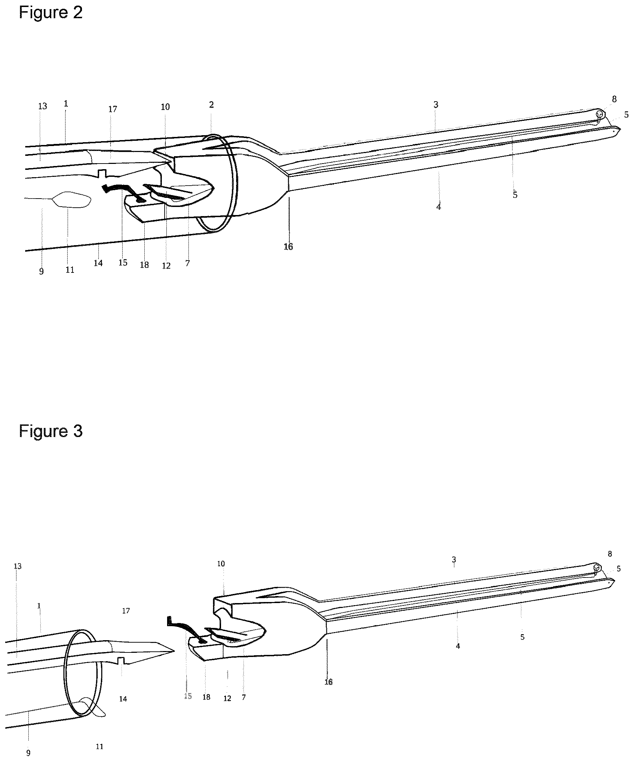

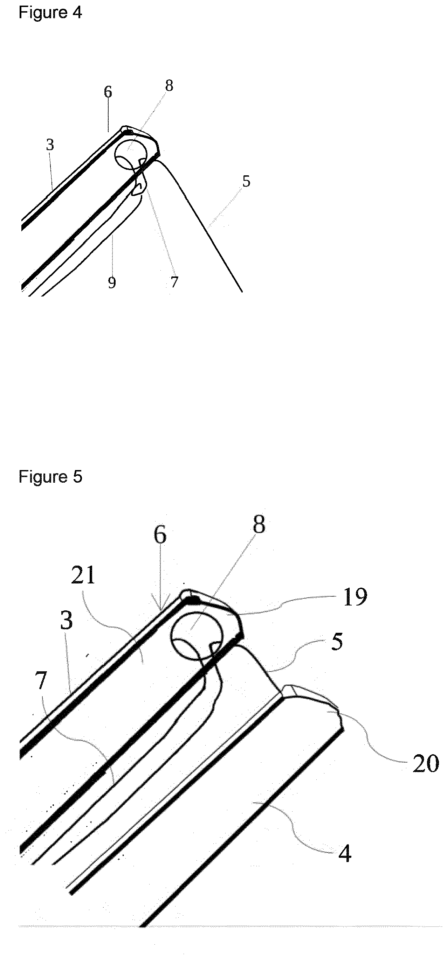

Dans la configuration ouverte [Fig 1], le premier 3 bras et second 4 bras du dispositif sont positionnés sur la zone de tissu cible à saisir. L'application d'une force prédéfinie sur l'actionneur (poignée de commande) permet le retrait du fil 9 souple de commande, entraînant avec lui la boucle 7 du fil 5 souple de serrage à travers la fente 8 dans l'extrémité distale du premier bras 3 [Fig 4]. L'application d'une force de traction au niveau de la poignée de commande entraîne le déplacement du fil 9 et de la boucle 7 du fil 5 souple de serrage jusqu'à sa fixation sur la saillie 12 au niveau de l'extrémité proximale du dispositif, permettant la fixation de la boucle 7 du fil 5 souple de serrage en position de traction. Cette traction provoque le rapprochement des extrémités distales des premier 3 et second 4 bras du dispositif permettant ainsi la fermeture ou la ligature du tissu cible. La poursuite de l'application de la force prédéfinie de la traction sur le fil 9 souple permet la libération de la boucle 11 de l'extrémité distale du fil 9 souple de commande de la saille 12. La liaison fragile 14 échoue à la poursuite de l'application de la force prédéfinie de la traction sur le fil 9 souple de commande, qui permet le passage de la boucle 11 du fil 9 souple de commande sur la saillie 15, ce qui provoque la séparation de l'extrémité distale du fil 13 rigide de la liaison fragile 14 [Fig 2]. L'application d'une force de poussée au niveau de la poignée de commande de l'extrémité proximale de l'élément souple 1 entraîne la sortie complète du dispositif de l'extrémité distale 2 de l'élément tubulaire flexible 1 et sa libération [Fig 3].

L'invention concerne selon un aspect un dispositif pour la fermeture ou la ligature de tissu au niveau de la paroi interne d'un organe creux comme le tube digestif ou d'autres lumières corporelles et organes creux caractérisés en ce qu'il comprend une partie proximale, (ladite partie proximale comprenant une fente 10 et une liaison fragile 14), un premier 3 et un second 4 bras d'une longueur prédéfinie, dont l'extrémité distale du second bras 4 est reliée par un fil 5 souple de serrage (de matière résorbable ou non résorbable) avec une longueur prédéfinie, un fil souple de commande 9 passant à travers ladite fente 10, Ledit fil 9 souple passe ensuite dans la boucle 7 du fil 5 souple de serrage (de matière résorbable ou non résorbable). Ledit fil 9 présente à son extrémité une boucle 11 de taille prédéfinie. La boucle 11 de l'extrémité du fil 9 souple est retenue sur une saillie 12 à l'extrémité proximale du dispositif, ladite saillie 12 permettant de retenir la boucle 7 et permettant la fixation du fil 5 souple de serrage en position de traction et un fil rigide de commande 13 positionné dans ladite liaison fragile 14. Le fil 13 rigide de commande est configuré pour déplacer le dispositif dans l'extrémité distale d'un élément tubulaire flexible 1 de telle sorte que les premier 3 et second 4 bras dudit dispositif puissent sortir de l'élément tubulaire flexible 1 permettant une position ouverte dudit dispositif et/ou que les premier 3 et second 4 bras dudit dispositif puissent être retirés dans l'extrémité distale de l'élément tubulaire flexible 1 permettant une position fermée dudit dispositif. La partie proximale du dispositif est fixée par l'extrémité distale d'un fil 13 rigide de commande positionnée dans une liaison fragile 14 sur une saillie 15 de forme courbée qui retient l'extrémité du fil 13 rigide dans la liaison 14 fragile qui échoue à la poursuite de l'application de la force prédéfinie de la traction sur le fil 9 souple de commande, qui permet le passage de la boucle 11 du fil 9 souple de commande sur la saillie 15, ce qui provoque la séparation de l'extrémité distale du fil 13 rigide de la liaison fragile 14 [Fig 2]. L'application d'une force de poussée au niveau de la poignée de commande de l'extrémité proximale de l'élément souple 1 entraîne la sortie complète du dispositif de l'extrémité distale 2 de l'élément tubulaire flexible 1 [Fig 3].

Les premier 3 et second 4 bras du dispositif présentent de préférence une angulation 16 [Fig 1] proximale prédéfinie permettant l'écartement des deux extrémités du premier 3 et du second 4 bras du dispositif une fois sortie de l'extrémité distale de l'élément tubulaire flexible 1 de telle sorte que dans la position ouverte dudit dispositif, les extrémités distales des premier 3 et second 4 bras sont séparées, permettant de positionner ledit dispositif sur la zone de tissu cible à saisir. L'extrémité distale du second bras 4 est reliée par un fil 5 souple de serrage avec une longueur prédéfinie, ledit fil 5 souple de serrage de matière prédéfinie résorbable ou non résorbable. L'autre extrémité dudit fil 5 souple de serrage est fixée sur le bord externe 6 de l'extrémité distale du premier bras 3, une boucle 7 dudit fil 5 souple de serrage passe du côté externe dans la fente 8 de l'extrémité distale du premier bras 3 [Fig 4].

En d'autres termes, l'invention concerne selon un aspect un dispositif pour la fermeture ou la ligature de tissu au niveau de la paroi interne d'un organe creux comme le tube digestif ou d'autres lumières corporelles et organes creux caractérisé en ce qu'il comprend un élément tubulaire 1 allongé et flexible, utilisé à travers un canal de travail d'un endoscope dont une extrémité proximale reste externe au corps, accessible à un utilisateur avec manette tandis qu'une extrémité distale 2 de l'élément tubulaire flexible 1 est insérée dans l'organe creux après son introduction dans le canal de travail de l'endoscope jusqu'à un emplacement adjacent au tissu cible à fermer ou à ligaturer, ledit dispositif comprenant aussi deux fils de commande, un fil 9 souple de commande et un fil rigide 13 de commande s'étendant à travers l'élément tubulaire flexible 1, les deux fils de commande 9, 13 étant couplés de manière amovible au dispositif, le dispositif comprenant un premier 3 et un second 4 bras chacun d'une longueur prédéfinie, l'extrémité distale du second bras 4 étant reliée par un fil souple de serrage 5 avec une longueur prédéfinie, l'autre extrémité du fil souple de serrage 5 étant fixée sur le bord externe 6 de l'extrémité distale du premier bras 3, une boucle 7 dudit fil souple de serrage 5 passant du coté externe dans une fente 8 de l'extrémité distale du premier bras 3, le fil souple de commande 9 passant dans une fente 10 au niveau de la partie proximale du dispositif, ledit fil souple de commande 9 passant ensuite dans la boucle 7 du fil souple de serrage 5, ledit fil souple de commande 9 présentant à son extrémité une boucle 11 de taille prédéfinie, la boucle 11 de l'extrémité du fil 9 souple étant retenue sur une saillie 12 à l'extrémité proximale du dispositif, la partie proximale 18 du dispositif étant fixée par l'extrémité distale 17 du fil rigide de commande 13 positionnée dans une liaison fragile 14, une saillie 15 de forme courbée retenant l'extrémité distale 17 du fil rigide de commande 13 dans la liaison fragile 14 qui échoue lorsque le dispositif est complètement sorti de l'extrémité distale 2 de l'élément tubulaire flexible 1 pour séparer le dispositif de l'attache du fil rigide de commande 13.

En d'autres termes encore, l'invention concerne selon un aspect un dispositif pour la fermeture ou la ligature d'un tissu cible au niveau de la paroi interne d'un organe creux comme le tube digestif ou d'autres lumières corporelles et organes creux. Le dispositif comprend ainsi :
- une partie proximale pourvue d'une première saillie 12 et d'une première fente 10,
- un premier bras 3 et un second bras 4 solidaires l'un de l'autre au niveau de ladite partie proximale 18, ledit premier bras 3 étant pourvu d'une première extrémité distale 19 et d'une deuxième fente 8, ledit second bras 3 étant pourvu d'une deuxième extrémité distale 20,
- un fil souple de serrage 5 reliant lesdits premier et second bras 3, 4 au niveau respectivement des première et deuxième extrémités distales 19, 20, ledit fil souple de serrage 5 formant une première boucle 7 passant, depuis un côté externe 6 du premier bas 3, dans la deuxième fente 8,
- un fil souple de commande 9 comprenant une deuxième boucle 11.

Ledit dispositif est conçu pour passer :
- d'une configuration ouverte (figure 1), où :
   ∘ lesdits première et deuxième extrémités distales 19, 20 sont écartées l'un de l'autre,
   ∘ le fil souple de commande 9 s'étend à travers ladite première fente 10, vers la première boucle 7, passe dans cette dernière, puis revient vers ladite partie proximale 18 pour se fixer à ladite première saillie 12 avec la deuxième boucle 11,
- à une configuration fermée (figures 2 et 3), après qu'une force de traction ait été exercée sur le fil souple de commande 9, où :
   ∘ ledit fil souple de commande 9 entraîne avec lui ladite première boucle 7 jusqu'à ladite première saillie 12, pour que cette dernière retienne ladite première boucle 7,
   ∘ ledit fil souple de commande 9 se libère de ladite première saillie 12,
   ∘ lesdits première et deuxième extrémités distales 19, 20 sont rapprochées l'une de l'autre grâce au fil de serrage 5, pour la fermeture ou la ligature du tissu cible.

En configuration ouverte, le dispositif est donc préférentiellement conçu pour qu'une partie au moins du fil souple de commande 9 s'étende le long du premier bras 3, à proximité de ce dernier et à l'écart du second bras 4. Ceci est notamment permis par le positionnement de la fente (plus précisément une lumière traversante ici) 8, au niveau de la première extrémité distale 19 du premier bas 3, par laquelle passe la première boucle 7 du fil de serrage 5. Ceci permet d'avoir un espace libre, dépourvu de fil (notamment de fil souple de commande 9, ou de fil souple de serrage 5), comme on le voit à la figure 1, cet espace libre étant destiné à accueillir le tissu cible à fermer ou à ligaturer. En configuration ouverte, la première boucle 7 se trouve donc avantageusement uniquement au voisinage de la première extrémité distale 19, car elle n'a pas été étirée et présente donc une taille réduite ou minimale (figures 1 et 4), tandis qu'en configuration fermée, la première boucle 7 s'étend avantageusement tout le long du premier bras 3 jusqu'à la partie proximale 18 (et plus précisément jusqu'à la première saillie 12), car elle a été étirée (par le fil souple de commande 9) et présente donc une taille agrandie ou maximale (figures 2 et 3, et particulièrement visible à la figure 5).

Ledit premier bras 3 comprenant de préférence, comme illustré, un côté ou bord interne 21 tourné vers ledit second bras 4 ainsi qu'un côté ou bord externe 6 à l'opposé dudit côté ou bord interne 21 et donc tourné vers l'extérieur. La première boucle 7 passe donc du côté ou bord externe 6 pour se retrouver face au côté ou bord interne 21, de préférence entre lesdits premier et second bras 3, 4, avantageusement au voisinage de la première extrémité distale 19 du premier bras 3 (au moins en configuration ouverte).

Selon un mode de réalisation avantageux et tel qu'illustré aux figures, le dispositif comprend un élément tubulaire 1 sensiblement allongé et flexible, destiné à être utilisé à travers un canal de travail d'un endoscope dont une extrémité proximale reste externe au corps et accessible à un utilisateur, par exemple avec une manette, l'élément tubulaire 1 comprenant ainsi une extrémité distale 2 destinée à être insérée dans l'organe creux après son introduction dans le canal de travail de l'endoscope jusqu'à un emplacement adjacent au tissu cible à fermer ou à ligaturer, ledit fil souple de commande 9 s'étendant à travers ledit élément tubulaire 1.

Le dispositif comprend de préférence un fil ou tige rigide de commande 13 s'étendant à travers ledit élément tubulaire 1 jusqu'à ladite partie proximale 18, ledit fil ou tige rigide de commande 13 étant conçu pour déplacer la partie proximale 18 au sein de l'élément tubulaire 1, par exemple par application d'une force de poussée ou de traction sur ledit fil ou tige rigide de commande 13, la poussée ou la traction étant de préférence réalisée relativement à la partie proximale 18 (poussée vers la partie proximale 18, traction à l'écart de celle-ci). Le fil ou tige rigide de commande 18 présente de préférence une tenue mécanique propre, et en particulier n'est pas flasque. Au contraire, les fils souples de commande 9 et de serrage 5 sont avantageusement flasques, sans tenue mécanique propre, comme illustré aux figures.

De manière préférentielle, le dispositif pour la fermeture ou la ligature d'un tissu cible est conçu pour passer :
- d'une configuration initiale (non illustrée), où :
   ∘ les premier et second bras 3, 4 sont positionnés au sein de l'élément tubulaire 1,
   ∘ lesdits première et deuxième extrémités distales 19, 20 sont rapprochées l'une de l'autre grâce à l'élément tubulaire 1 entourant lesdits premier et second bras 3, 4,
   ∘ les fils souples de serrage 5 et de commande 9 sont sensiblement comme dans la configuration ouverte,
- à la configuration ouverte, où de surcroît les premier et second bras 3, 4 sont positionnés hors de l'élément tubulaire 1.

Le passage de la configuration initiale à la configuration ouverte se fait de préférence par l'application d'une force de poussée sur le fil ou tige rigide de commande 13 au sein de l'élément tubulaire 1, ladite force de poussée étant transmise par le fil ou tige rigide de commande 13 à ladite partie proximale 18.

De manière préférentielle, le fil ou tige rigide de commande 13 est pourvue d'une quatrième extrémité distale 17 conçue pour interagir avec ladite partie proximale 18, et d'une troisième extrémité proximale (non illustrée, mais de préférence située hors de l'organe creux) permettant à un utilisateur de manipuler le déplacement dudit fil ou tige rigide de commande 13 au sein de l'élément tubulaire 1, et notamment de pousser ledit fil ou tige rigide de commande 13.

De préférence, comme illustré à la figure 1 notamment, ladite quatrième extrémité distale 17 est en butée contre la partie proximale 18, notamment en configuration initiale et/ou en configuration ouverte.

Selon un mode de réalisation particulier, ledit fil ou tige rigide de commande 13 comprend, au niveau de ladite quatrième extrémité distale 17, un moyen de liaison fragile 14, et en ce que ladite partie proximale 18 est pourvue d'une deuxième saillie 15, de préférence distincte de ladite première saillie 12, ledit moyen de liaison fragile 14 et ladite deuxième saillie 15 étant conçus pour :
- en configuration initiale et/ou en configuration ouverte, être attachés ensemble, de préférence mécaniquement,
- en configuration fermée, être désolidarisés l'un de l'autre, de sorte que l'élément tubulaire 1, le fil souple de commande 9 et le fil ou tige rigide de commande 13 forment un premier ensemble (à gauche sur la figure 3), ledit premier ensemble étant alors désolidarisé d'un second ensemble (à droite sur la figure 3) comprenant au moins la partie proximale 18, les premier et second bras 3, 4, et le fil souple de serrage 15.

Le dispositif est conçu avantageusement pour que l'application d'une force de poussée au niveau de la troisième extrémité proximale du fil ou tige rigide de commande 13 entraîne la sortie complète du second ensemble hors de l'élément tubulaire 1, comme illustré à la figure 3 (plus précisément, entre la figure 2 et 3, il y a la force de poussée susmentionnée qui a été appliquée).

Ledit second ensemble reste avantageusement à l'endroit de la ligature ou de la fermeture du tissu, tandis que le premier ensemble est retiré hors de l'organe creux et plus généralement hors du corps du patient.

De manière avantageuse, ladite partie proximale 18 comprend un moyen de déformation élastique de rappel 16 permettant de positionner automatiquement les premier et second bras 3, 4 à une angulation prédéfinie l'un relativement à l'autre. Les première et deuxième extrémités distales 19, 20 sont donc éloignées l'une de l'autre, dès lors que lesdits premier et second bras 3, 4 sont sortis hors de l'élément tubulaire 1 et que le fil souple de serrage 5 n'est pas tendu ou tiré par le fil souple de commande 9, en particulier en configuration ouverte, ce qui permet de positionner lesdits premier et second bras 3, 4 au niveau du tissu cible à fermer ou à ligaturer. En d'autres termes, le moyen de déformation élastique de rappel 16 permet d'écarter automatiquement lesdits premier et second bras 3, 4, dès lors qu'ils ne sont pas contraints et rapprochés l'un de l'autre par une autre force, par exemple exercée par le fil de serrage 5 (configuration fermée, figures 2 et 3), ou par une paroi interne de l'élément tubulaire 1 (configuration initiale, non illustrée ici).

De manière préférentielle, comme illustré aux figures 1 et 4, ladite première boucle 7 est positionnée sensiblement entre lesdits premier et second bras 3, 4.

En résumé, de manière particulièrement avantageuse, lesdits premier et second bras 3, 4 forment une pince refermable, l'ouverture de la pince étant réalisée automatiquement, en configuration ouverte, à l'aide d'un moyen de rappel, par exemple un moyen de déformation élastique de rappel 16, tandis que la fermeture de la pince est réalisée, en configuration fermée, par le fil souple de serrage 5 qui est maintenu en tension pour conserver lesdites première et deuxième extrémités distales 19, 20 à proximité l'une de l'autre.

### DESCRIPTION BRÈVE DES DESSINS

La figure 1 est une vue du dispositif monobloc selon un mode de réalisation de la présente invention, en configuration ouverte.
La figure 2 est une vue en perspective du dispositif monobloc après séparation du dispositif selon un mode de réalisation de la présente invention, en configuration fermée, avec la partie proximale 18 encore en partie dans l'élément tubulaire 1.
La figure 3 est une vue en perspective du dispositif monobloc après séparation du dispositif de l'élément tubulaire 1 selon un mode de réalisation de la présente invention, et donc en configuration fermée, la partie proximale 18 étant donc complètement sortie de l'élément tubulaire flexible 1.
La figure 4 est une vue rapprochée de la première extrémité distale 19 du premier bras 3, en configuration ouverte (et éventuellement en configuration initiale).
La figure 5 est une vue rapprochée des première et deuxième extrémités distales 19, 20 appartenant respectivement au premier bras 3 et au second bras 4, en configuration fermée.

### Légende :

(1) Élément tubulaire flexible ;
(2) Extrémité distale de l'élément tubulaire flexible 1, dite troisième extrémité distale ;
(3) Premier bras ;
(4) Deuxième (Second) bras ;
(5) Fil souple de serrage ;
(6) Bord externe du premier bras 3, au niveau de la première extrémité distale 19 du premier bras (3) ;
(7) Première boucle du fil souple de serrage 5 ;
(8) Fente (ou lumière traversante) de la première extrémité distale 9 du premier bras 3 ;
(9) Fil souple de commande ;
(10) fente (ou lumière traversante) au niveau de la partie proximale 18 du dispositif ;
(11) Deuxième boucle de l'extrémité distale 9 du fil souple de commande 9 ;
(12) Première saillie, au niveau de l'extrémité (ou partie) proximale 18 du dispositif ;
(13) Fil (ou tige) rigide de commande ;
(14) Liaison fragile ou moyen de liaison fragile, en l'occurrence ici une encoche ;
(15) Deuxième saillie, de préférence de forme courbée ;
(16) Angulation proximale prédéfinie, ou moyen de déformation élastique de rappel de la partie proximale 18, permettant l'écartement (de préférence prédéfini), en configuration ouverte, des première et deuxième extrémités distales 19, 20 respectivement du premier 3 et du second 4 bras ;
(17) Extrémité distale du fil rigide de commande 13, dite quatrième extrémité distale ;
(18) Partie (ou extrémité) proximale du dispositif ;
(19) Première extrémité distale du premier bras 3 ;
(20) Deuxième extrémité distale du second bras 4 ;
(21) côté ou bord interne du premier bras 3.

## Revendications

1. Dispositif pour la fermeture ou la ligature d'un tissu cible au niveau de la paroi interne d'un organe creux comme le tube digestif ou d'autres lumières corporelles et organes creux, **caractérisé en ce qu'**il comprend :
- une partie proximale pourvue d'une première saillie (12) et d'une première fente (10),
- un premier bras (3) et un second bras (4) solidaires l'un de l'autre au niveau de ladite partie proximale (18), ledit premier bras (3) étant pourvu d'une première extrémité distale (19) et d'une deuxième fente (8), ledit second bras (3) étant pourvu d'une deuxième extrémité distale (20),
- un fil souple de serrage (5) reliant lesdits premier et second bras (3, 4) au niveau respectivement des première et deuxième extrémités distales (19, 20), ledit fil souple de serrage (5) formant une première boucle (7) passant, depuis un côté externe (6) du premier bas (3), dans la deuxième fente (8),
- un fil souple de commande (9) comprenant une deuxième boucle (11),
ledit dispositif étant conçu pour passer :
- d'une configuration ouverte, où :
∘ lesdits première et deuxième extrémités distales (19, 20) sont écartées l'un de l'autre,
∘ le fil souple de commande (9) s'étend à travers ladite première fente (10), vers la première boucle (7), passe dans cette dernière, puis revient vers ladite partie proximale (18) pour se fixer à ladite première saillie (12) avec la deuxième boucle (11),
- à une configuration fermée, après qu'une force de traction ait été exercée sur le fil souple de commande (9), où :
∘ ledit fil souple de commande (9) entraîne avec lui ladite première boucle (7) jusqu'à ladite première saillie (12), pour que cette dernière retienne ladite première boucle (7),
∘ ledit fil souple de commande (9) se libère de ladite première saillie (12),
∘ lesdits première et deuxième extrémités distales (19, 20) sont rapprochées l'une de l'autre grâce au fil de serrage (5), pour la fermeture ou la ligature du tissu cible.

2. Dispositif pour la fermeture ou la ligature d'un tissu cible selon la revendication précédente, **caractérisé en ce qu'**il comprend un élément tubulaire (1) sensiblement allongé et flexible, destiné à être utilisé à travers un canal de travail d'un endoscope dont une extrémité proximale reste externe au corps et accessible à un utilisateur, par exemple avec une manette, l'élément tubulaire (1) comprenant ainsi une troisième extrémité distale (2) destinée à être insérée dans l'organe creux après son introduction dans le canal de travail de l'endoscope jusqu'à un emplacement adjacent au tissu cible à fermer ou à ligaturer, ledit fil souple de commande (9) s'étendant à travers ledit élément tubulaire (1).

3. Dispositif pour la fermeture ou la ligature d'un tissu cible selon la revendication précédente, **caractérisé en ce qu'**il comprend un fil ou tige rigide de commande (13) s'étendant à travers ledit élément tubulaire (1) jusqu'à ladite partie proximale (18), ledit fil ou tige rigide de commande (13) étant conçu pour déplacer la partie proximale (18) au sein de l'élément tubulaire (1).

4. Dispositif pour la fermeture ou la ligature d'un tissu cible selon la revendication précédente, **caractérisé en ce qu'**il est conçu pour passer :
- d'une configuration initiale, où :
∘ les premier et second bras (3, 4) sont positionnés au sein de l'élément tubulaire (1),
∘ lesdits première et deuxième extrémités distales (19, 20) sont rapprochées l'une de l'autre grâce à l'élément tubulaire (1) entourant lesdits premier et second bras (3, 4),
∘ les fils souples de serrage (5) et de commande (9) sont sensiblement comme dans la configuration ouverte,
- à la configuration ouverte, où de surcroît les premier et second bras (3, 4) sont positionnés hors de l'élément tubulaire (1),
le passage de la configuration initiale à la configuration ouverte se faisant par l'application d'une force de poussée sur le fil ou tige rigide de commande (13) au sein de l'élément tubulaire (1), ladite force de poussée étant transmise par le fil ou tige rigide de commande (13) à ladite partie proximale (18).

5. Dispositif pour la fermeture ou la ligature d'un tissu cible selon la revendication 3 ou 4, **caractérisé en ce que** le fil ou tige rigide de commande (13) est pourvue d'une quatrième extrémité distale (17) conçue pour interagir avec ladite partie proximale (18), et d'une troisième extrémité proximale permettant à un utilisateur de manipuler le déplacement dudit fil ou tige rigide de commande (13) au sein de l'élément tubulaire (1), et notamment de pousser ledit fil ou tige rigide de commande (13).

6. Dispositif pour la fermeture ou la ligature d'un tissu cible selon la revendication précédente, **caractérisé en ce que** ladite quatrième extrémité distale (17) est en butée contre la partie proximale (18), notamment en configuration initiale et/ou en configuration ouverte.

7. Dispositif pour la fermeture ou la ligature d'un tissu cible selon la revendication 5 ou 6, **caractérisé en ce que** ledit fil ou tige rigide de commande (13) comprend, au niveau de ladite quatrième extrémité distale (17), un moyen de liaison fragile (14), et **en ce que** ladite partie proximale (18) est pourvue d'une deuxième saillie (15), de préférence distincte de ladite première saillie (12), ledit moyen de liaison fragile (14) et ladite deuxième saillie (15) étant conçus pour :
- en configuration initiale et/ou en configuration ouverte, être attachés ensemble, de préférence mécaniquement,
- en configuration fermée, être désolidarisés l'un de l'autre, de sorte que l'élément tubulaire (1), le fil souple de commande (9) et le fil ou tige rigide de commande (13) forment un premier ensemble, ledit premier ensemble étant alors désolidarisé d'un second ensemble comprenant au moins la partie proximale (18), les premier et second bras (3, 4), et le fil souple de serrage (15).

8. Dispositif pour la fermeture ou la ligature d'un tissu cible selon la revendication précédente, **caractérisé en ce qu'**il est conçu pour que l'application d'une force de poussée au niveau de la troisième extrémité proximale du fil ou tige rigide de commande (13) entraîne la sortie complète du second ensemble hors de l'élément tubulaire (1).

9. Dispositif selon l'une quelconque des revendications 2 à 8, **caractérisé en ce que** ladite partie proximale (18) comprend un moyen de déformation élastique de rappel (16) permettant de positionner automatiquement les premier et second bras (3, 4) à une angulation prédéfinie l'un relativement à l'autre, les première et deuxième extrémités distales (19, 20) étant donc éloignées l'une de l'autre, dès lors que lesdits premier et second bras (3, 4) sont sortis hors de l'élément tubulaire (1) et que le fil souple de serrage (5) n'est pas tendu ou tiré par le fil souple de commande (9), en particulier en configuration ouverte, ce qui permet de positionner lesdits premier et second bras (3, 4) au niveau du tissu cible à fermer ou à ligaturer.

10. Dispositif selon l'une quelconque des revendications précédentes, **caractérisé en ce que** ladite première boucle (7) est positionnée sensiblement entre lesdits premier et second bras (3, 4).

11. Dispositif selon l'une quelconque des revendications précédentes, **caractérisé en ce que** lesdits premier et second bras (3, 4) forment une pince refermable, l'ouverture de la pince étant réalisée automatiquement, en configuration ouverte, à l'aide d'un moyen de rappel, par exemple un moyen de déformation élastique de rappel (16), tandis que la fermeture de la pince est réalisée, en configuration fermée, par le fil souple de serrage (5) qui est maintenu en tension pour conserver lesdites première et deuxième extrémités distales (19, 20) à proximité l'une de l'autre.

## Patentansprüche

1. Vorrichtung zum Schließen oder Abbinden eines Zielgewebes an der inneren Wand eines Hohlorgans, wie zum Beispiel des Verdauungstraktes oder anderer Körper- und Organlumen, **dadurch gekennzeichnet, dass** sie umfasst:
• einen proximalen Teil, der mit einem ersten Vorsprung (12) und einem ersten Schlitz (10) versehen ist,
• einen ersten Arm (3) und einen zweiten Arm (4), die am proximalen Teil (18) miteinander verbunden sind, wobei der erste Arm (3) mit einem ersten distalen Ende (19) und einem zweiten Schlitz (8) versehen ist und der zweite Arm (4) ein zweites distales Ende (20) aufweist,
• einen flexiblen Zugfaden (5), der den ersten und den zweiten Arm (3, 4) an den jeweiligen distalen Enden (19, 20) verbindet und eine erste Schlaufe (7) bildet, die von einer äußeren Seite (6) des ersten Arms (3) in den zweiten Schlitz (8) eintritt,
• einen flexiblen Steuerfaden (9) mit einer zweiten Schlaufe (11), wobei die Vorrichtung so ausgelegt ist, dass sie von einer offenen Konfiguration, in der:
• das erste und das zweite distale Ende (19, 20) voneinander beabstandet sind,
• der Steuerfaden (9) durch den ersten Schlitz (10) zur ersten Schlaufe (7) verläuft, diese durchläuft und dann zum proximalen Teil (18) zurückkehrt, um mit der zweiten Schlaufe (11) am ersten Vorsprung (12) befestigt zu werden, in eine geschlossene Konfiguration übergeht, nachdem eine Zugkraft auf den Steuerfaden (9) ausgeübt wurde, wobei:
• der Steuerfaden (9) die erste Schlaufe (7) bis zum ersten Vorsprung (12) zieht, sodass dieser die erste Schlaufe (7) hält,
• der Steuerfaden (9) sich vom ersten Vorsprung (12) löst,
• das erste und das zweite distale Ende (19, 20) durch den Zugfaden (5) aufeinander zugezogen werden, um das Zielgewebe zu schließen oder abzubinden.

2. Vorrichtung zum Schließen oder Abbinden eines Zielgewebes gemäß dem vorangegangenen Anspruch, **dadurch gekennzeichnet, dass** sie ein im Wesentlichen langgestrecktes und flexibles Rohrelement (1) umfasst, das zur Verwendung durch einen Arbeitskanal eines Endoskops bestimmt ist, dessen proximales Ende außerhalb des Körpers verbleibt und für einen Benutzer zugänglich ist, zum Beispiel mit einem Hebel, wobei das Rohrelement (1) ein drittes distales Ende (2) umfasst, das in das Hohlorgan eingeführt werden soll, nachdem es in den Arbeitskanal des Endoskops eingebracht wurde, zu einer Position neben dem zu schließenden oder abzubindenden Zielgewebe, wobei der Steuerfaden (9) durch das Rohrelement (1) verläuft.

3. Vorrichtung zum Schließen oder Abbinden eines Zielgewebes gemäß dem vorangegangenen Anspruch, **dadurch gekennzeichnet, dass** sie einen starren Steuerdraht oder -stab (13) umfasst, der durch das Rohrelement (1) bis zum proximalen Teil (18) verläuft, wobei der Steuerdraht oder -stab (13) so gestaltet ist, dass er den proximalen Teil (18) innerhalb des Rohrelements (1) bewegt.

4. Vorrichtung zum Schließen oder Abbinden eines Zielgewebes gemäß dem vorangegangenen Anspruch, **dadurch gekennzeichnet, dass** sie so gestaltet ist, dass sie von einer Anfangskonfiguration, in der:
• der erste und der zweite Arm (3, 4) im Rohrelement (1) positioniert sind,
• die ersten und zweiten distalen Enden (19, 20) durch das umgebende Rohrelement (1) nahe beieinander liegen,
• die flexiblen Zug- und Steuerfäden (5, 9) im Wesentlichen wie in der offenen Konfiguration positioniert sind, in die offene Konfiguration übergeht, in der die ersten und zweiten Arme (3, 4) außerhalb des Rohrelements (1) positioniert sind, wobei der Übergang von der Anfangskonfiguration in die offene Konfiguration durch:
• das Ausüben einer Schubkraft auf den starren Steuerdraht oder -stab (13) innerhalb des Rohrelements (1) erfolgt, wobei diese Schubkraft durch den Steuerdraht oder - stab (13) auf den proximalen Teil (18) übertragen wird.

5. Vorrichtung zum Schließen oder Abbinden eines Zielgewebes gemäß den Ansprüchen 3 oder 4, **dadurch gekennzeichnet, dass** der starre Steuerdraht oder -stab (13) mit einem vierten distalen Ende (17) versehen ist, das zur Interaktion mit dem proximalen Teil (18) ausgelegt ist, und mit einem dritten proximalen Ende, das es einem Benutzer ermöglicht, den Steuerdraht oder -stab (13) innerhalb des Rohrelements (1) zu bewegen, insbesondere den Steuerdraht oder -stab (13) zu schieben.

6. Vorrichtung zum Schließen oder Abbinden eines Zielgewebes gemäß dem vorangegangenen Anspruch, **dadurch gekennzeichnet, dass** das vierte distale Ende (17) gegen den proximalen Teil (18) anliegt, insbesondere in der Anfangs- und/oder offenen Konfiguration.

7. Vorrichtung zum Schließen oder Abbinden eines Zielgewebes gemäß den Ansprüchen 5 oder 6, **dadurch gekennzeichnet, dass** der Steuerdraht oder -stab (13) an dem vierten distalen Ende (17) ein brüchiges Verbindungsmittel (14) umfasst und der proximale Teil (18) mit einem zweiten Vorsprung (15) versehen ist, vorzugsweise getrennt von dem ersten Vorsprung (12), wobei das brüchige Verbindungsmittel (14) und der zweite Vorsprung (15) so gestaltet sind, dass sie:
• in der Anfangs- und/oder offenen Konfiguration miteinander verbunden sind, vorzugsweise mechanisch,
• in der geschlossenen Konfiguration voneinander gelöst werden, sodass das Rohrelement (1), der Steuerfaden (9) und der Steuerdraht oder -stab (13) eine erste Einheit bilden, wobei die erste Einheit dann von einer zweiten Einheit getrennt ist, die mindestens den proximalen Teil (18), die ersten und zweiten Arme (3, 4) und den Zugfaden (5) umfasst.

8. Vorrichtung zum Schließen oder Abbinden eines Zielgewebes gemäß dem vorangegangenen Anspruch, **dadurch gekennzeichnet, dass** sie so gestaltet ist, dass durch das Ausüben einer Schubkraft auf das dritte proximale Ende des Steuerdrahts oder -stabs (13) die vollständige Ausbringung der zweiten Einheit aus dem Rohrelement (1) bewirkt wird.

9. Vorrichtung gemäß einem der Ansprüche 2 bis 8, **dadurch gekennzeichnet, dass** der proximale Teil (18) ein elastisches Rückstellmittel (16) umfasst, das dazu dient, die ersten und zweiten Arme (3, 4) in einem voreingestellten Winkel zueinander zu positionieren, wobei die ersten und zweiten distalen Enden (19, 20) voneinander entfernt sind, sobald die ersten und zweiten Arme (3, 4) außerhalb des Rohrelements (1) positioniert sind und der Zugfaden (5) nicht durch den Steuerfaden (9) gespannt oder gezogen wird, insbesondere in der offenen Konfiguration, was es ermöglicht, die ersten und zweiten Arme (3, 4) auf das Zielgewebe auszurichten, das geschlossen oder abgebunden werden soll.

10. Vorrichtung gemäß einem der vorangegangenen Ansprüche, **dadurch gekennzeichnet, dass** die erste Schlaufe (7) im Wesentlichen zwischen den ersten und zweiten Armen (3, 4) positioniert ist.

11. Vorrichtung gemäß einem der vorangegangenen Ansprüche, **dadurch gekennzeichnet, dass** die ersten und zweiten Arme (3, 4) eine schließbare Zange bilden, wobei die Öffnung der Zange in der offenen Konfiguration automatisch durch ein Rückstellmittel, beispielsweise ein elastisches Rückstellverformungsmittel (16), erfolgt, während das Schließen der Zange in der geschlossenen Konfiguration durch den Zugfaden (5) erfolgt, der gespannt bleibt, um die ersten und zweiten distalen Enden (19, 20) nahe beieinander zu halten.

## Claims

1. Device for closing or ligating a target tissue at the inner wall of a hollow organ, such as the digestive tract or other body lumens and hollow organs, **characterized in that** it comprises:
• a proximal part provided with a first protrusion (12) and a first slot (10),
• a first arm (3) and a second arm (4) fixed to each other at said proximal part (18), said first arm (3) being provided with a first distal end (19) and a second slot (8), said second arm (4) being provided with a second distal end (20),
• a flexible tightening thread (5) connecting said first and second arms (3, 4) at their respective first and second distal ends (19, 20), said flexible tightening thread (5) forming a first loop (7) passing from an outer side (6) of the first arm (3) into the second slot (8),
• a flexible control thread (9) comprising a second loop (11), wherein said device is designed to move:
• from an open configuration, wherein:
• said first and second distal ends (19, 20) are spaced apart from each other,
• the control thread (9) extends through said first slot (10) toward the first loop (7), passes through it, and then returns to said proximal part (18) to be fixed to said first protrusion (12) with the second loop (11),
• to a closed configuration, after a pulling force has been exerted on the control thread (9), wherein:
• the control thread (9) pulls along said first loop (7) up to said first protrusion (12), so that the latter retains said first loop (7),
• the control thread (9) releases from said first protrusion (12),
• said first and second distal ends (19, 20) are drawn toward each other by the tightening thread (5), for closing or ligating the target tissue.

2. Device for closing or ligating a target tissue according to the preceding claim, **characterized in that** it comprises a substantially elongated and flexible tubular element (1) intended to be used through a working channel of an endoscope, one proximal end of which remains external to the body and accessible to a user, for example with a lever, the tubular element (1) thus comprising a third distal end (2) intended to be inserted into the hollow organ after its introduction into the endoscope's working channel to a location adjacent to the target tissue to be closed or ligated, said control thread (9) extending through said tubular element (1).

3. Device for closing or ligating a target tissue according to the preceding claim, **characterized in that** it comprises a rigid control wire or rod (13) extending through said tubular element (1) to said proximal part (18), said rigid control wire or rod (13) being designed to move the proximal part (18) within the tubular element (1).

4. Device for closing or ligating a target tissue according to the preceding claim, **characterized in that** it is designed to transition:
• from an initial configuration, wherein:
• the first and second arms (3, 4) are positioned within the tubular element (1),
• said first and second distal ends (19, 20) are close to each other by the surrounding tubular element (1),
• the flexible tightening and control threads (5, 9) are in substantially the same configuration as in the open configuration,
• to the open configuration, wherein the first and second arms (3, 4) are positioned outside the tubular element (1), the transition from the initial to the open configuration being achieved by:
• applying a pushing force on the rigid control wire or rod (13) within the tubular element (1), said pushing force being transmitted by the rigid control wire or rod (13) to said proximal part (18).

5. Device for closing or ligating a target tissue according to claims 3 or 4, **characterized in that** the rigid control wire or rod (13) is provided with a fourth distal end (17) designed to interact with said proximal part (18), and a third proximal end allowing a user to manipulate the movement of said rigid control wire or rod (13) within the tubular element (1), specifically to push said rigid control wire or rod (13).

6. Device for closing or ligating a target tissue according to the preceding claim, **characterized in that** said fourth distal end (17) abuts against the proximal part (18), particularly in the initial and/or open configuration.

7. Device for closing or ligating a target tissue according to claims 5 or 6, **characterized in that** said rigid control wire or rod (13) comprises, at said fourth distal end (17), a fragile connecting means (14), and **in that** said proximal part (18) is provided with a second protrusion (15), preferably separate from said first protrusion (12), said fragile connecting means (14) and said second protrusion (15) being designed to:
• in the initial and/or open configuration, be attached together, preferably mechanically,
• in the closed configuration, be detached from each other, so that the tubular element (1), the control thread (9), and the rigid control wire or rod (13) form a first assembly, said first assembly being then detached from a second assembly comprising at least the proximal part (18), the first and second arms (3, 4), and the tightening thread (5).

8. Device for closing or ligating a target tissue according to the preceding claim, **characterized in that** it is designed such that applying a pushing force on the third proximal end of the rigid control wire or rod (13) causes the complete ejection of the second assembly from the tubular element (1).

9. Device according to any one of claims 2 to 8, **characterized in that** said proximal part (18) includes an elastic return deformation means (16) for automatically positioning the first and second arms (3, 4) at a predefined angle relative to each other, the first and second distal ends (19, 20) thus being spaced apart, once the first and second arms (3, 4) are outside the tubular element (1) and the tightening thread (5) is neither taut nor pulled by the control thread (9), particularly in the open configuration, allowing positioning of the first and second arms (3, 4) at the target tissue to be closed or ligated.

10. Device according to any one of the preceding claims, **characterized in that** said first loop (7) is positioned substantially between said first and second arms (3, 4).

11. Device according to any one of the preceding claims, **characterized in that** the first and second arms (3, 4) form a closable clamp, the clamp opening being achieved automatically in the open configuration using a return means, for example an elastic return deformation means (16), while the clamp closure is achieved in the closed configuration by the tightening thread (5) which is held in tension to keep said first and second distal ends (19, 20) close to each other.
